# EUROPEAN PATENT SPECIFICATION

(11) **EP 1 294 311 B1**
(45) Date of publication and mention of the grant of the patent: **04.01.2006**
(21) Application number: 01935692.2
(22) Date of filing: 18.05.2001
(51) Int. Cl.: A61F 2/06

(54) **BILIARY STENT AND METHOD OF MAKING IT**
GALLENGANGSTENT UND VERFAHREN ZU SEINER HERSTELLUNG
STENT BILIAIRE ET PROCEDE DE FABRICATION

(30) Priority: 19.05.2000 US 205744 P
(43) Date of publication of application: 26.03.2003
(73) Proprietor: CONMED Endoscopic Technologies, Inc., Billerica, MA 01821 (US)
(72) Inventor: AZNOIAN, Harold, M., N. Andover, MA 01845 (US); RAIJMAN, Isaac, Bell Aire, TX 77401 (US)
(74) Representative: HOFFMANN EITLE
(86) International application number: PCT/US2001/016132
(87) International publication number: WO 2001/089419

(56) References cited:
- EP-A- 0 451 996
- WO-A-00/18322
- US-A- 3 105 492
- US-A- 5 129 910
- US-A- 5 609 624
- US-A- 5 910 170
- US-A- 5 984 965

## Description

### Field of the Invention

This invention relates to stents and, in particular, to stents adapted for use in the biliary tract.

Biliary stents, for many years, have been made in the form of a polymer tube that can be advanced on a delivery catheter through an endoscope and into the bile duct where it is deployed. The tubular stent is selected to be sufficiently strong to resist collapse to maintain an open lumen through which digestive liquids can flow into the digestive tract. Among the desirable features of such a stent is that it be longitudinally flexible to be advanced along a path that may include sharp bends. The stent also should maintain its intended position within the bile duct without migrating from that position.

### Background of the Invention

In US 5,984,965 a stent for placement in a body passage and maintaining the patency of the body passage is disclosed. The stent may be constructed of a bioresorbable material, and comprises an elongate flexible body and a ridge defining a series of spaced apart barriers for transporting masses and fluid along said body. In WO 00/18322 a stent according to the preamble of claim 1 is disclosed.

Polymeric tubular stents typically have been placed with a catheter-like device that includes telescoping inner and outer tubes, with the stent being mounted on the distal end of the inner tube and the distal end of the outer tube being in engagement with the proximal end of the stent After the stent has been advanced and manipulated into the intended deployment site in the duct, the outer tube is maintained in its position while the inner tube is retracted, thereby leaving the stent within the biliary tract. Generally, such stents are provided with a retention member at each of the ends of the stent. Among the more common retention devices is the provision of one or more (four to eight are common) retention tabs formed by making an oblique slit along the length of the tube. Each slit defines a tab and enables the tab to project slightly radially outwardly of the outer surface of the tube to engage the luminal surface of the biliary duct to prevent migration. The tabs at the opposite ends of the stent extend toward the middle of the stent as well as radially outward.

The openings defined by the tab-forming skives may provide access to the interior of the stent of cellular or other material that may tend to develop into an obstruction tending to restrict flow through the stent. Also among the difficulties with prior polymeric stents is that in some cases the physician may not be able to push the stent through a constriction in the duct. It is among the general objects of the invention to provide a polymeric stent that displays a combination of significant longitudinal flexibility to fadlitate its placement and, significant hoop strength to resist collapse of the stent. It is also among the objects of the invention to provide a method for making a stent.

### Summary of the Invention

The invention is defined in claim 1 and 13. Optional and preferred features are recited in the dependent claims.

The stent is formed from a tube of relatively stiff thermoplastic polymer to include ridges and valleys along its outer surface. The ridges and valleys may be helical and may form a thread-like configuration. The ridges and valleys are formed by thermoplastic deformation of the outer surface of the tube. The dimensions of the ridges and valleys can be varied to provide stents with different characteristics. The proximal and distal ends of the stent are preferably not provided with valleys or ridges. The distal end may be tapered to facilitate its entry into the biliary tract. Additionally, the distal end of the stent, which will serve as an inlet for biliary liquids, may have an elongate shape to provide a wider mouth for entry of such liquids. The device may be placed by pushing it to the desired location in the biliary tree, as is presently done otherwise, the stent can be rotated so that the helical ridges and valleys can serve as threads to advance the stent through a biliary stricture. The ridges engage the walls of the duct to secure the stent in place.

It is among the general objects of the invention to provide an improved stent, particularly for use in the biliary tract Also among the objects of the invention are to provide a stent for use in the biliary tract in which the stent is easily fabricated from a polymeric material and embodies a construction that enables the characteristics of the stent to be varied easily; to provide a stent that is very flexible yet in which the flexibility can be controiied during manufacture without changing the general structure of the stent and; to provide a stent that can be advanced into place by pushing it into place or by threading it through a biliary stricture.

### Description of the Accompanying Drawings

The foregoing and other objects and advantages of the invention will be appreciated more fully from the following description thereof, with reference to the accompanying drawings wherein:
FIG. 1 is a side view of a stent in accordance with the invention in which portions of the stent are broken away;
FIG. 2 is an enlarged illustration of the presently preferred embodiment of the proximal end of the stent;
FIG. 3 is a side view of the distal end of the stent;
FIGS. 4 and 5 are top and side views of the thermo forming tool in engagement with the polymer tubing during formation of the stent;
FIG. 6 is an illustration, as seen along the axis of the starting tube during formation illustrating the manner in which the forming tool may press the starting tube against the outer surface of an undersized mandrel passing through the starting tube; and
FIG. 7 is an illustration of the distal end of an embodiment of the invention seen along the line 7-7 of FIG.1.
FIG. 8 is a side sectional view of a stent delivery device.

### Description of the Illustrative Embodiments

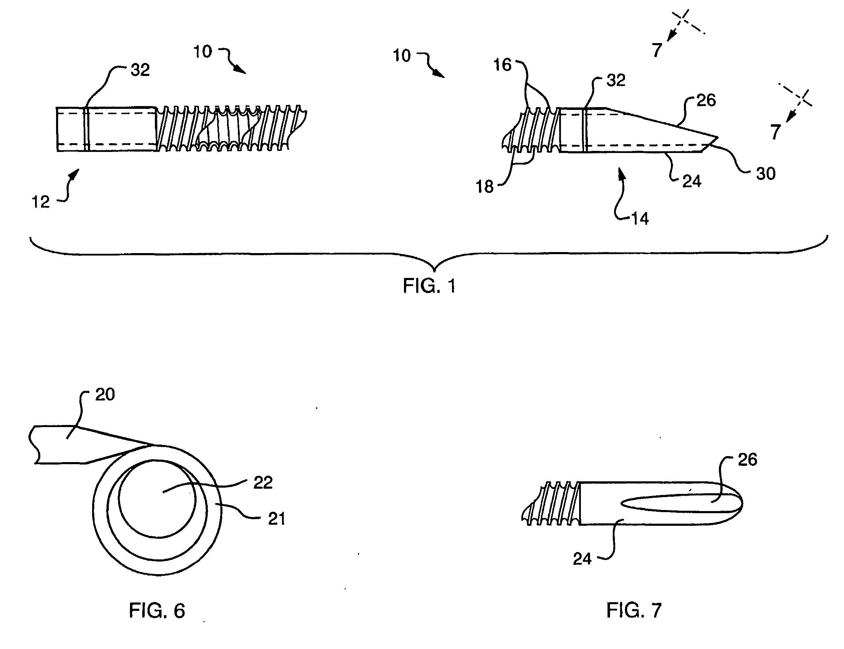

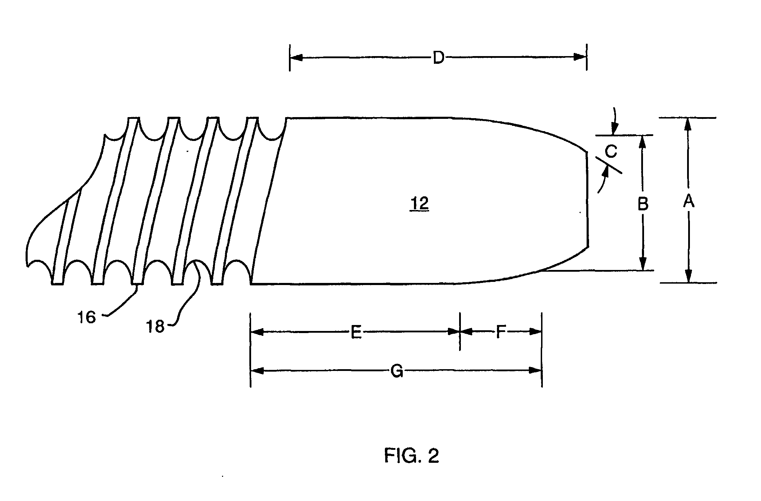

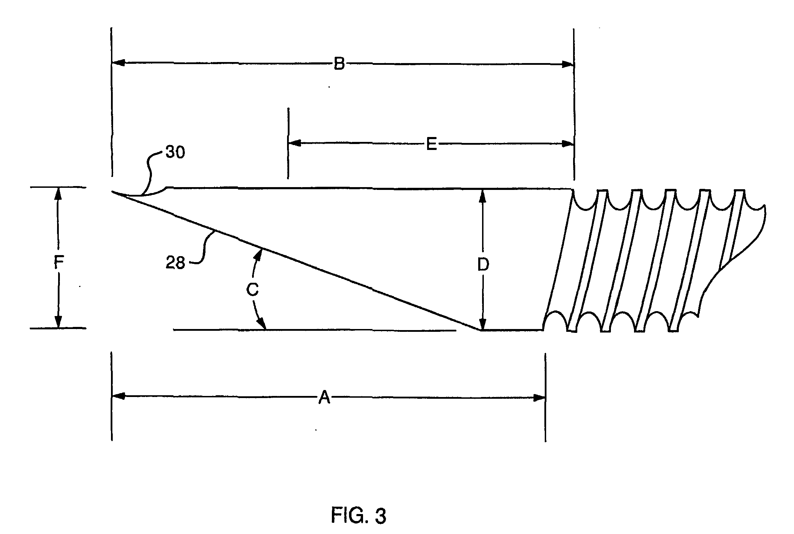

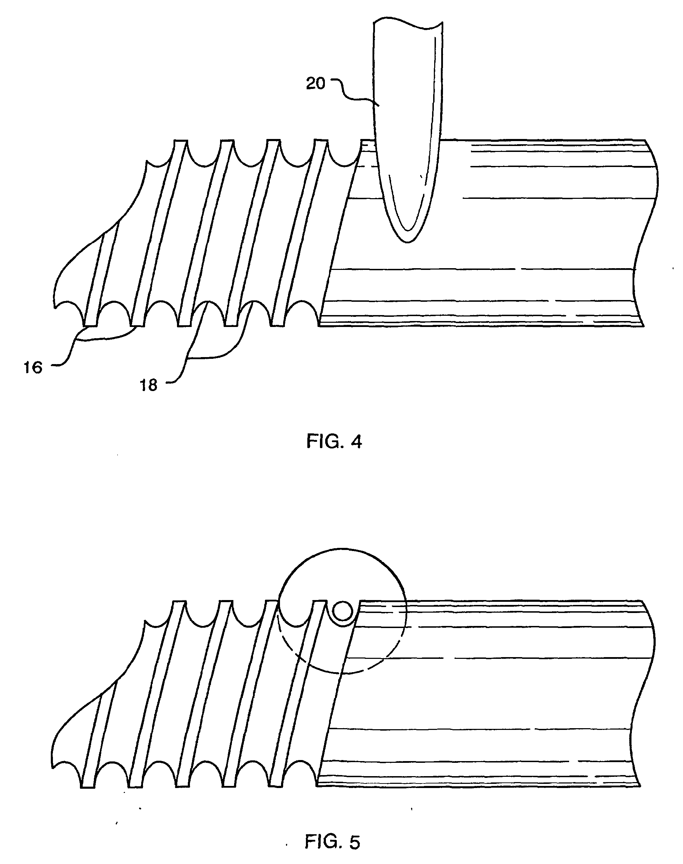

FIG. 1 Illustrates, in side view, an embodiment of a stent 10 having a proximal end 12 and a distal end 14. The stent is formed from a tube of a polymer, commercially available from Vctrex under the trade designation PEEK. The polymer is a polyetheretherketone, a linear aromatic semi-crystalline polymer. By way of example, for use as a biliary stent, the PEEK tubing may be of the order of 4 to 15 centimetres long having an outer diameter of between about 1.651 to 3.6322 mm (5 to 11 French, or .065 inches to 0.143 inches, respectively). The wall thickness of the tubing may be of the order of about 127 µm (about 0.005 inches). The PEEK material is thermoplastic and is formed from its extruded tubular configuration to that illustrated in FIG. 1 in which at least a portion of the length of the tube defines circumferentially extending external ridges 16 alternating with valleys 18. Preferably the ridges are formed in a helical, thread-like pattern.

The ridges 16 and valleys 18 are formed by applying a heated tool against the outer surface of the starting tube while rotating the tube and advancing the tool along the length of the rotating tube. FIGS. 4 and 5 illustrate, diagrammatically, a simplified technique for making the stent in which a generally conically shaped heated tip 20, as might be mounted on the end of a soldering iron, is applied to the external surface of the tubing while the tubing is rotated. The heat and pressure of the thermo forming tool 20 causes the thermoplastic tubing to become flowable in the localized region of the tool, thereby forming the valleys and ridges in the outer surface of the tube. We have found that it is possible to form the stent so that the inner surface of the tube also includes valleys and ridges corresponding to those on the outer surface by initially mounting the starting tube on a mandrel that has an outer diameter smaller than the inner diameter of the PEEK tubing. By way of example, we have found that mounting tubing 21 on a cylindrical mandrel 22 having an outer diameter about 254 µm (about 0.010 inches) smaller than the inner diameter of the starting tube (see FIG. 6), the configuration of inner and outer ridges and valleys results. It is believed that the inner surface of the tube also forms the ridges and valleys as a consequence of the localized cooling of the polymer immediately behind the axially advancing thermofomning tool. The clearance between the outer diameter of the mandrel and the inner diameter of the tubing is believed to contribute to the ability of the tubing to cool and form in that fashion.

The configuration of the thermo forming tool and the penetration depth to which the tool is applied to the outer surface of the PEEK tube can be varied to vary the characteristics of the stent. Additionally, the speed the tube is rotated and/or the speed the tool is advanced along the length of the tube can also be varied to alter the characteristics of the stent Deeper grooves 18 may result in a thinner wall having greater flexibility. Similarly, the pitch of the ridges 16 can be varied to vary the characteristics of the stent. As will be understood, increasing the number of threads per unit length of tube will increase the ability to finely adjust the placement of the stent via rotation while decreasing the number of threads per unit length of tube will decrease the ability to finely adjust the placement of the stent. The thread density can thus be adjusted to the particular characteristics of the luminal wall engaged by the stent. By way of example, a relatively rigid luminal wall will allow the use of a densely threaded stent. A relatively flexible or pliable luminal wall will require a stent with less dense and larger threading to ensure the preferably helical threads positively engage the wall to allow for advancement of the stent via rotation.

Preferably the proximal end of the starting tube will not have been formed to include the ridges and valleys The proximal end of the stent may be configured and dimensioned as indicated in FIG. 2, in which the end is somewhat radiused or rounded. The rounding may be effected in any number of ways, such as by placing a mandrel having rounded ends within the tube and heating the tube proximal end while rotating the tube to form the rounded end. Another possible approach is to round over the proximal end with a solder tip held against the end while rotating the tube. A yet further approach is to use a knife held at an angle against the proximal end while rotating the tube. The distal end may be provided with a similarly fashioned tip, configured and dimensioned as indicated in FIG. 3. Alternately, it may be preferable to provide a modified tip 24 at the distal end 14 that has a generally tapered configuration to facilitate its entry through the papilla and into the biliary duct. The distal tip also may be configured to provide a distal opening 26 (FIG. 7) that is elongated and may be generally elliptical in shape. The elongated distal opening may facilitate entry of biliary liquids into the stent by providing an inlet opening larger than the transverse cross section of the lumen. To that end, the distal end of the tube is formed with an oblique cut 28 to expose an elongate inlet opening 26. The distal tip also may be beveled or otherwise tapered at its opposite side as shown at 30.

The stent may be provided with marker bands 32 at one or both of the distal and proximal ends. The marker bands 32 may be formed from gold or other suitably radiopaque material. Circular grooves may be thermoformed in either or both of the ends 12, 14 and the radiopaque marker bands may be secured within those grooves. The ridges 16 and grooves 18 may be formed along substantially the full length of the stent or may be formed only along selected segments, for example, adjacent the ends of the stent, leaving the mid portion in its original tubular configuration. Conversely, only the mid portion may be provided with the ridges and valleys. Stiil further, ridges 16 and grooves 18 can be placed in selected sporadic groups along the length of the stent to achieve stiffness and flexibility characteristics tailored to particular needs or particular anatomy. The valleys and ridges can be made to be circumferentially segmented portions as opposed to being complete annular rings or completely helical by intermittently withdrawing and applying the thermoforming tool. Additionally, the pitch of the ridges and the depth of the valleys can be varied along any segment or along the full length of the stent in order to provide varied flexing characteristics for the stent.

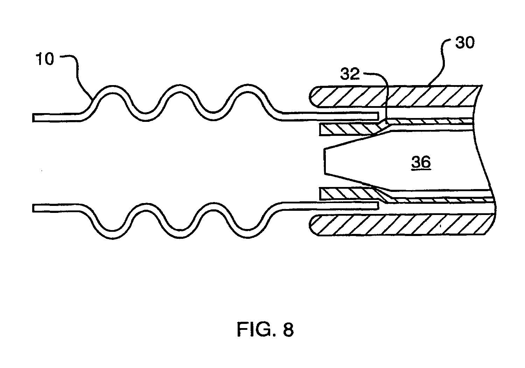

The stent may be placed in the biliary duct either by the conventional pushing technique or by mounting it on a rotatable delivery catheter having a stent engaging member engageable with the proximal end of the stent FIG. 8 shows a catheter 30 with a stent engaging member 32 in the form of an expandable collar that is received within the proximal end of the stent 10 and expanded securely against the inner luminal surface at the proximal end with wedge 36. Stent 10 is advanced to a selected site in the biliary tract with catheter 30. Wedge 36 is then proximally retracted to release the frictional engagement of engaging member 32 from stent 10. Stent 10 is then released using the conventional pushing technique. In an alternate embodiment, as the stent is advanced into the biliary duct, the an alternate delivery device (not shown) may be rotated to facilitate entry of the stent through an obstructed portion of the duct to that end, it is preferred that the ridges and valleys be formed to define a helical path that will enable the stent to advance, in screw-like fashion through the obstruction. The stent may be released from the delivery device after it has been deployed in the desired location.

The ridges may engage the inner surface of the duct to secure the stent in place. Additionally, when the valley 18 is continuous, as defined by a helical path, it may be possible for biliary liquids to flow between the outer surface of the stent and the wall of the biliary duct as well as through the stent itself. To perform the latter function, valleys 18 have to be formed with sufficient pitch, depth and/or angle to maintain an open channel since it is anticipated the duct wall will partially hemiate into the valley. Another benefit of having a relatively deep valley is that such a configuration is expected to enhance the mechanical engagement of the stent to the duct wall.

It should be understood that the foregoing description of the invention is intended merely to be illustrative thereof and that modifications and other embodiments may be apparent to those who are skilled in the art.

## Claims

1. A tubular biliary stent (10) adapted to be advanced on a delivery catheter along the bile duct in the form of a polymer tube having an external surface, at least part of which is defined by circumferentially extending ridges (16) and valleys (18), and wherein
the ridges and valleys are formed within the wall thickness of the polymer tube so as to render the stent flexible enough that it can be advanced on a delivery catheter along the bile duct **characterized in that**
the stent is formed from a polymer that is a polyetheretherketone (PEEK).

2. The stent of claim 1, wherein the ridges and valleys are disposed helically along the outer surface of the stent.

3. The stent of claim 2, wherein the ridges and valleys extend at least partially along the full length of the stent.

4. The stent of claim 3, wherein the ridges and valleys are disposed in partial circumferential segments.

5. The stent of claim 4, wherein the ridges and valley disposed in partial circumferential segments have a helical orientation relative to a longitudinal axis of the stent.

6. The stent of any of the preceding claims further comprising a proximal end (12) and a distal end (14), wherein the ends are free of ridges and valleys.

7. The stent of any one of claims 1 to 5 further comprising a proximal end and a distal end, wherein the ridges and valleys are disposed on at least one of the ends.

8. The stent of any one of claims 1 to 5 further comprising a proximal end and a distal end, wherein the ridges and valleys are disposed in close proximity to at least one end.

9. The stent of any one of the preceding claims further comprising at least one marker band (32) located adjacent to at least one end of the stent.

10. The stent of any one of the preceding claims further comprising a proximal end and a distal end, wherein at least one of the ends has an elongate inlet opening.

11. The stent of claim 10, wherein the distal end of the stent is bevelled.

12. The stent of any one of the preceding claims further comprising an inner surface, at least part of which is defined by circumferentially extending ridges and valleys.

13. A method for making a biliary stent adapted to be advanced on a delivery catheter along the bile duct, comprising the steps of
providing a thermoplastic tube made of polyetheretherketone,
rotating the thermoplastic tube;
placing a thermoforming tool (20) against a sidewall of the tube; and
forming circumferentially extending ridges and valleys about the external surface of the tube.

14. The method of claim 13 further comprising:
advancing the thermoforming tool along the length of the tube to form ridges and valleys that define a helical configuration.

15. The method of claim 13 or 14 further comprising:
supporting the tube on a mandrel having an outside diameter less than an inside diameter of the tube.

16. The method of claim 13, 14 or 15 further comprising:
cyclically applying and removing the thermoforming tool while the tube is rotated.

17. The method of anyone of claims 13 to 16 further comprising supporting the tube on a mandrel and heat molding the outer surface of the tube while on the mandrel.

## Patentansprüche

1. Rohrförmiger Gallen-Stent (10), der auf einem Abgabekatheter entlang der Gallengänge vorwärts bewegbar ist, in der Form eines Polymerrohrs mit einer äußeren Oberfläche, von der zumindest ein Teil durch sich am Umfang erstreckende Kämme (16) und Täler (18) definiert ist,
und wobei die Kämme und Täler in der Wanddicke des Polymerrohrs ausgebildet sind, um den Stent flexibel genug zu machen, dass er auf einem Abgabekatheter entlang der Gallengänge vorwärts bewegt werden kann,
**dadurch gekennzeichnet, dass**
der Stent aus einem Polymer ausgebildet ist, das ein Polyetheretherketon (PEEK) ist.

2. Stent nach Anspruch 1, bei dem die Kämme und Täler spiralförmig entlang der äußeren Oberfläche des Stents angeordnet sind.

3. Stent nach Anspruch 2, bei dem sich die Kämme und Täler zumindest teilweise entlang der ganzen Länge des Stents erstrecken.

4. Stent nach Anspruch 3, bei dem die Kämme und Täler in Teilumfangssegmenten angeordnet sind.

5. Stent nach Anspruch 4, bei dem die Kämme und Täler, die in Teilumfangssegmenten angeordnet sind, eine schraubenförmige Orientierung relativ zu einer Längsachse des Stents aufweisen.

6. Stent nach einem der vorhergehenden Ansprüche, ferner mit einem proximalen Ende (12) und einem distalen Ende (14), wobei die Enden frei von Kämmen und Tälern sind.

7. Stent nach einem der Ansprüche 1 bis 5, ferner mit einem proximalen Ende und einem distalen Ende, wobei die Kämme und Täler an zumindest einem der Enden angeordnet sind.

8. Stent nach einem der Ansprüche 1 bis 5, ferner mit einem proximalen Ende und einem distalen Ende, wobei die Kämme und Täler in dichter Nähe zu zumindest einem Ende angeordnet sind.

9. Stent nach einem der vorhergehenden Ansprüche, ferner mit zumindest einem Markierband (32), das sich an zumindest ein Ende des Stents angrenzend befindet.

10. Stent nach einem der vorhergehenden Ansprüche, ferner mit einem proximalen Ende und einem distalen Ende, wobei zumindest eines der Enden eine längliche Einlassöffnung aufweist.

11. Stent nach Anspruch 10, bei dem das distale Ende des Stents abgeschrägt ist.

12. Stent nach einem der vorhergehenden Ansprüche, ferner mit einer inneren Oberfläche, von der zumindest ein Teil durch sich am Umfang erstreckende Kämme und Täler definiert ist.

13. Verfahren zur Herstellung eines Gallen-Stents, der auf einem Abgabekatheter entlang der Gallengänge vorwärts bewegbar ist, die folgenden Schritte umfassend:
Vorsehen eines thermoplastischen Rohrs, das aus Polyetheretherketon hergestellt ist;
Drehen des thermoplastischen Rohrs;
Platzieren eines Thermoformungswerkzeugs (20) gegen eine Seitenwand des Rohrs; und
Ausbilden von sich am Umfang erstreckenden Kämmen und Tälern um die äußere Oberfläche des Rohrs.

14. Verfahren nach Anspruch 13, ferner mit:
Vorwärtsbewegen des Thermoformungswerkzeugs entlang der Länge des Rohrs, um Kämme und Täler auszubilden, die eine schraubenförmige Konfiguration definieren.

15. Verfahren nach Anspruch 13 oder 14, ferner mit:
Stützen des Rohrs auf einem Dorn mit einem Außenseitendurchmesser, der geringer als ein Innenseitendurchmesser des Rohrs ist.

16. Verfahren nach Anspruch 13, 14 oder 15, ferner mit:
zyklischem Aufbringen und Entfernen des Thermoformungswerkzeugs während das Rohr gedreht wird.

17. Verfahren nach einem der Ansprüche 13 bis 16, ferner mit:
Stützen des Rohrs auf einem Dorn, und Wärme-Formbilden der äußeren Oberfläche des Rohrs während es auf dem Dorn ist.

## Revendications

1. Endoprothèse biliaire tubulaire (10) susceptible d'être introduite, sur un cathéter de pose, le long du canal cholédoque, se présentant sous la forme d'un tube en polymère présentant une surface externe, dont au moins une partie est définie par des crêtes (16) et des sillons (18) s'étendant sur la circonférence, les crètes et sillons étant formés dans l'épaisseur de paroi du tube en polymère afin de rendre l'endoprothèse suffisamment flexible pour être introduite, sur un cathéter de pose, le long du canal cholédoque, **caractérisée en ce que** l'endoprothèse est constituée en un polymère est qui est du polyétheréthercétone (PEEK).

2. Endoprothèse selon la revendication 1, dans laquelle les crêtes et sillons sont disposés de façon hélicoïdale sur la surface externe de l'endoprothése.

3. Endoprothèse selon la revendication 2, dans laquelle les crêtes et sillons s'étendent, au moins partiellement, sur toute la longueur de l'endoprothèse.

4. Endoprothèse selon la revendication 3, dans laquelle les crêtes et sillons sont disposés en segments partiels circonférentiels.

5. Endoprothèse selon la revendication 4, dans laquelle les crêtes et sillons disposés en segments partiels circonférentiels présentent une orientation hélicoïdale par rapport à un axe longitudinal de l'endoprothèse.

6. Endoprothèse selon l'une quelconque des revendications précédentes, comprenant, en outre, une extrémité proximale (12) et une extrémité distale (14), les extrémités étant dépourvues de crêtes et sillons.

7. Endoprothèse selon l'une quelconque des revendications 1 à 5, comprenant, en outre, une extrémité proximale et une extrémité distale, les crêtes et sillons étant disposés sur au moins l'une des extrémités.

8. Endoprothèse selon l'une quelconque des revendications 1 à 5. comprenant, en outre, une extrémité proximale et une extrémité distale, les crêtes et sillons étant disposés tout près d'au moins une extrémité.

9. Endoprothèse selon l'une quelconque des revendications précédentes, comprenant, en outre, au moins une bande servant de marqueur (32) adjacente à au moins une extrémité de l'endoprothèse.

10. Endoprothèse selon l'une quelconque des revendications précédentes; comprenant, en outre, une extrémité proximale et une extrémité distale, au moins l'une des extrémités présentant une ouverture d'entrée allongée.

11. Endoprothèse selon la revendication 10, dans laquelle l'extrémité distale de l'endoprothèse est taillée en biseau.

12. Endoprothèse selon l'une quelconque des revendications précédentes, comprenant, en outre, une surface interne, dont au moins une partie est définie par des crêtes et sillons s'étendant sur la circonférence.

13. Procédé de fabrication d'une endoprothèse biliaire susceptible d'être introduite, sur un cathéter de pose, le long du canal cholédoque, comprenant les étapes qui consistent à :
prévoir un tube thermoplastique constitué en polyétheréthercétone,
faire tourner le tube thermoplastique ;
placer l'outil de thermoformage (20) contre une paroi latérale du tube ; et
former des crêtes et sillons, s'étendant sur la circonférence, autour de la surface externe du tube.

14. Procédé selon la revendication 13, comprenant, en outre , l'étape qui consiste à :
faire avancer l'outil de thermoformage sur la longueur du tube afin de former des crêtes et des sillons définissant une configuration hélicoïdale.

15. Procédé selon la revendication 13 ou 14, comprenant, en outre, l'étape qui consiste à :
faire reposer le tube sur un mandrin présentant un diamètre extérieur inférieur à un diamètre intérieur du tube.

16. Procédé selon la revendication 13, 14 ou 15 comprenant, en outre, l'étape qui consiste à :
appliquer et retirer, de façon cyclique, l'outil de thermoformage pendant que le tube est amené à tourner.

17. Procédé selon l'une quelconque des revendications 13 à 16, comprenant, en outre, l'étape qui consiste à faire reposer le tube sur un mandrin et à mouler à chaud la surface externe du tube pendant qu'il se trouve sur le mandrin.
